# EUROPEAN PATENT SPECIFICATION

(11) **EP 1 322 243 B1**
(45) Date of publication and mention of the grant of the patent: **26.10.2005**
(21) Application number: 01966938.1
(22) Date of filing: 27.09.2001
(51) Int. Cl.: A61B 17/66

(54) **MAXILLARY DISTRACTOR**
DISTRAKTIONSVORRICHTUNG FÜR DEN OBERKIEFER
SEPARATEUR MAXILLAIRE

(30) Priority: 04.10.2000 US 237519 P
(43) Date of publication of application: 02.07.2003
(73) Proprietor: Synthes AG Chur, 7002 Chur (CH)
(72) Inventor: SELLERS, Timothy M., Wayne, PA 19087 (US); NOON, John M., Paoli, PA 19301 (US); HAAG, René, Berwyn, PA 19312 (US)
(74) Representative: Lusuardi, Werther Giovanni
(86) International application number: PCT/CH2001/000583
(87) International publication number: WO 2002/028298

(56) References cited:
- WO-A-00/56235
- WO-A-98/16163
- US-A- 5 885 289
- US-A- 5 885 290

## Description

The present invention relates to an orthopedic system and, more particularly, to an improved orthopedic system wherein the device is used intra-orally in a patient to achieve a change in the position of the maxilla (upper jawbone) in relation to the zygoma (cheekbones).

A variety of orthopedic devices, including bone reduction and distraction devices, are known in the art. Reduction and distraction devices (commonly referred to as reducers and distractors), are used to gradually adjust the relative orientation and spacing of the bone parts on opposing sides of a bone repair site. As used herein, "bone repair site" refers to any bone region which is bounded on opposing sides by relatively healthy bone regions to which orthopedic devices can be secured, such as an osteotomy (cutting of a bone) or a fracture.

Reducers and distractors typically consist of transcutaneous pins or screws secured in the bone on either side of the bone repair site together with a mechanism which allows controlled incremental adjustment of the distance between parts of the device on opposing sides of the bone repair site. Typically, distractors are used to perform distraction osteogenesis (the formation of bone). This procedure was perfected by the Russian orthopedic doctor, Gavriel Ilizarov. A typical procedure of this type involves at most an osteotomy completely separating the bone into two segments, or at least an incision of the cortical portion of the bone. Then, the bone segments on either side of the osteotomy (or the medullary or cancellous portion of the bone on either side of the incision) may be expanded. This gradual separation allows new bone to form in the osteotomy void. The distraction phase is followed by a consolidation phase, during which the distractor is held fixed, and the new bone growth gains strength. Following the consolidation phase, the distractor is removed from the patient.

One area in which distraction techniques are used is in treating patients diagnosed with maxillary hypoplasia (underdevelopment of the maxilla, or upper jawbone). One particular patient population with this condition is cleft-lip and -palate patients. The key reason for utilizing maxillary distraction to treat these patients is in the ability to successfully overcome the substantial soft tissue forces found in the maxillary region of these patients. Cleft-lip and -palate patients usually undergo surgery to correct their soft tissue deformities in early infancy. These procedures involve a great deal of soft tissue dissection, and leave the patient with significant scar tissue surrounding their maxillary region. As a result of the reduced elasticity of the scar tissue as compared to regular soft tissue, the maxilla is very often restricted from normal growth and can be very difficult to advance using conventional orthognathic surgery (surgery relating to treatment of the malpositioning of bones of the jaw). Maxillary distraction thus allows the tensile forces of the scar tissue to be overcome, and a greater advancement distance to be achieved, with a clinically supported expectation of a lesser degree of relapse (undesired movement of maxilla back towards its original position after treatment is finished).

An additional patient population that can take advantage of maxillary distraction is non-cleft palate patients having an A-P (Anterior-Posterior) maxillary deficiency of large magnitude. Typically, orthognathic procedures involving maxillary advancements are limited in the magnitude of the advancement of the maxilla due to the elastic properties of the surrounding soft tissues. Also, the larger advancements are more likely to require a bone graft to the site to ensure the long-term stability of the advancement. Using distraction for maxillary advancements can eliminate the magnitude limitations as well as the need for grafting for these patients.

Another benefit of performing maxillary distraction on cleft-lip and -palate patients is the ability to treat the maxillary hypoplastic patients at a younger age than with conventional orthognathic surgery. Early treatment of skeletal deformities has been gaining in popularity among craniofacial surgeons as a means of minimizing the negative psychosocial impact that craniofacial deformities have on children. Also, some surgeons believe that early correction of skeletal deformities can reduce the residual impact on surrounding tissues and structures, thus improving the overall result for the patient. See, for example, Steven Cohen, M.D., F.A.C.S., "Midface Distraction," Perspectives in Plastic Surgery, Vol. 11, No. 1.

However, available devices that can be used for maxillary distraction have external "halo-style" fixators that attach to the skull and to the maxilla by way of surgical wires affixed to an intra-oral appliance. One such known halo system is the KLS-Martin RED (Rigid External Distraction) system. Such a high profile external device is unsightly, and the psychosocial effects of wearing an external device is a major concern, especially with younger patients. An external device is also more subject to bumps and snags than one which is completely located within a patient's body. Accordingly, there is a need in the art to provide a device that can be used intra-orally to reliably perform distraction or reduction of the maxilla.

Furthermore, the known external fixators involve a large number of component parts and accordingly are complicated to install and adjust. Accordingly, there is a need in the art to provide a device that can be used to perform distraction or reduction of the maxilla that has a relatively low part count, and is simple both to install and adjust.

From US-Patent 5,885,290 GUERRERO ET AL. a bone distraction device is known which is fixed by screws to a sectioned bone to facilitate multidirectional repositioning of the jaw bone. This device can be attached between the maxilla and the zygoma as specified in the preamble of claim 1. The fixation occurs by means of rigid wires having free ends in the form of fork-like anchors. Therefore this known device has the drawback that once it is assembled it does not allow a modification of the orientation of its components without deformation of the latter.

The orthopedic system of the present invention includes an orthopedic device for modifying the separation between the maxilla and the zygoma. The device is capable of being placed intra-orally and is attached to the maxilla and the zygoma in a patient.

The orthopedic device in the system is a distractor or distraction assembly. The system has at least two bone anchors, which are in the form of footplates, each with at least two screw holes adapted to receive a bone screw to anchor the footplate to the bone.

One of the footplates may also be attachable to one or more constructs secured to the patient. The orientation of the anchors may be modified and fixed in relation to each other.

An orthopedic system for modifying the distance between the maxilla and the zygoma of a patient according to the present invention comprises distal and proximal footplates, and an orthopedic device. The distal footplate is designed for subcutaneous implantation and attachment to the zygoma, with the footplate having a coupling surface and screw holes adapted to receive screws. The proximal footplate is designed for subcutaneous implantation and attachment to the maxilla, with the footplate having a coupling surface and screw holes adapted to receive screws. The orthopedic device has a longitudinal axis and allows for modification of the distance along the longitudinal axis between the footplates once the device is implanted in patient, with the device further allowing modification of the orientation of the footplates in relation to each other before implantation. The entire system is located intra-orally in the patient.

The orthopedic device is a distractor or distraction assembly. The distal footplate may be attached to the patient's zygoma and the proximal footplate may be attached to the patient's maxilla. In a method not forming part of the invention the distal footplate may be attached to the patient's zygoma and the proximal footplate may be attached to a construct, with the construct being mechanically coupled to the patient's teeth. When the footplates are engaged with the orthopedic device, the coupling surface of the distal footplate may be substantially orthogonal to the longitudinal axis of the device and the coupling surface of the proximal footplate may be substantially parallel to the longitudinal axis of the device.

In accordance with the present invention, the orthopedic device may comprise a lead screw, an inner sleeve, and an outer sleeve. The lead screw has external threading. The inner sleeve has an internal thread that engages the external thread of the lead screw, an external axial slot, and a distal footplate engagement portion, with the distal footplate engagement portion being coupled to the distal footplate. The outer sleeve has a slot-engaging portion and a proximal footplate engagement portion, the proximal footplate engagement portion being coupled to the proximal footplate. The lead screw and the outer sleeve are joined such that only relative rotational movement about the longitudinal axis is allowed, and the inner sleeve interacts with the lead screw and outer sleeve such that rotation of the lead screw is translated into linear movement of the inner sleeve in relation to the outer sleeve and lead screw.

The slot-engaging portion of the outer sleeve may be an indentation in the distal end of the outer sleeve, extending radially inward to interact with the external axial slot of the inner sleeve. The slot-engaging portion of the outer sleeve may also be a pin inserted through the distal end of the outer sleeve, extending radially inward to interact with the external axial slot of the inner sleeve.

The lead screw may be joined to the outer sleeve by a collar that rotates in conjunction with the lead screw. The collar may be joined to the lead screw by means of a pin inserted through both parts. The collar may have a visual reference mark to aid in converting angular displacement of the lead screw to linear advancement of the outer sleeve in relation to the inner sleeve. The visual reference mark may be an indentation in the surface of the collar, or it may be imprinted onto the surface of the collar.

The distal footplate may have a bore with an internal diameter matching that of the inner sleeve, and may be attached to the inner sleeve by pressing the two together and inserting a pin through both parts. The proximal footplate may have a bore with internal thread matching the external thread of the outer sleeve, and may be attached to the outer sleeve of the device by screwing the footplate onto the outer sleeve. There may also be a locking nut engaging the external thread of the outer sleeve, with the proximal footplate being locked into place by tightening the nut against it. When the footplates are engaged with the orthopedic device, the coupling surface of the distal footplate may be substantially orthogonal to the longitudinal axis of the device and the coupling surface of the proximal footplate may substantially parallel to the longitudinal axis of the device.

The footplates may be made of stainless steel, titanium, or titanium alloy. Alternatively, they may be composed of a bioresorbable material. They may also be detachable from the distraction assembly.

Also disclosed is a method (which does not form part of the invention) for modifying the separation between the maxilla and the zygoma of a patient. Generally, the method comprises the following steps. Incisions are made to access to the maxilla and zygoma. An intra-oral orthopedic device is mechanically coupled to the maxilla and zygoma. The orthopedic device is used to perform distraction osteogenesis. The orthopedic device is then removed from the patient, and the incisions are closed.

In this method, the orthopedic device may be a distractor. The step of mechanically coupling the distractor to the patient may consist of attaching the orthopedic device to the patient's bone, or alternatively, attaching the orthopedic device to a construct, which is attached to the teeth of the patient.

More specifically, the method for advancing the separation of the maxilla from the zygoma of a patient according to the present invention comprising the following steps. An incision is made to expose the maxilla and zygoma. A first orthopedic device comprising distal and proximal footplates and a distraction assembly is selected. The distal footplate is attached to the zygoma using screws. The proximal footplate is mechanically coupled to the maxilla using screws. A second orthopedic device is then attached to the other side of the patient using the same steps. An osteotomy is performed to separate the maxilla from the zygoma, and then the incisions are closed, leaving at least a portion of the distraction assembly of each device exposed. The distraction procedure is performed, using the distraction assemblies to increase the separation of the maxilla from the zygoma. After a period of time allowed for consolidation has passed, the devices are removed.

In the above method, the proximal footplate may be attached to the patient's maxilla. It may also be attached to a construct which is mechanically coupled to the patient's maxilla, which then requires attaching the construct to the maxilla.

The osteotomy performed in the above method may be a LeFort I-type osteotomy. The method may also include, before the step of performing the osteotomy, initially implanting the orthopedic devices temporarily in order to plan the osteotomy in view of the device locations. The orthopedic devices are then removed, and then replaced and reattached after performing the osteotomy.

Alternatively, the footplates and the screws used are made of a bioresorbable material, and instead of removing the entire device from the patient, the footplates, while still being attached to the patient, are detached from the distraction assembly before closing the incisions, leaving the footplates and screws to be absorbed into the body.

The features and advantages of the present invention will become more readily apparent from the following detailed description of the invention in which like elements are labeled similarly and in which:
Fig. 1 is a perspective view of an embodiment of the present orthopedic system adapted for use on the maxilla, illustrating a distractor attached to the maxilla and zygoma;
   Fig. 2 is a perspective view of a distractor of the system as illustrated in Fig. 1;
Fig. 3A and 3B are side views of the distraction assembly of the distractor illustrated in Fig. 2, in partial section and partial elevation view, showing the distractor at various stages of advancement;
Fig. 4 is a top plan view of the inner sleeve and the outer sleeve of the distractor illustrated in Fig. 2;
Fig. 5 is a sectional side view of the inner sleeve and the outer sleeve of Fig. 4 interacting with each other;
Fig. 6 is a perspective view of the proximal footplate illustrated in Fig. 2;
Fig. 7 is a perspective view of the distal footplate illustrated in Fig. 2;
Figs. 8A to 8E are side views showing the successive steps in the assembly of the device illustrated in Fig. 2; and
Fig. 9A and 9B are side and front views, respectively, of the system as illustrated in Fig. 2, when used in an alternative method of treatment.

The orthopedic device of the present invention is discussed herein with reference to a preferred embodiment adapted to be used in a linear distraction of the maxilla from the zygoma.

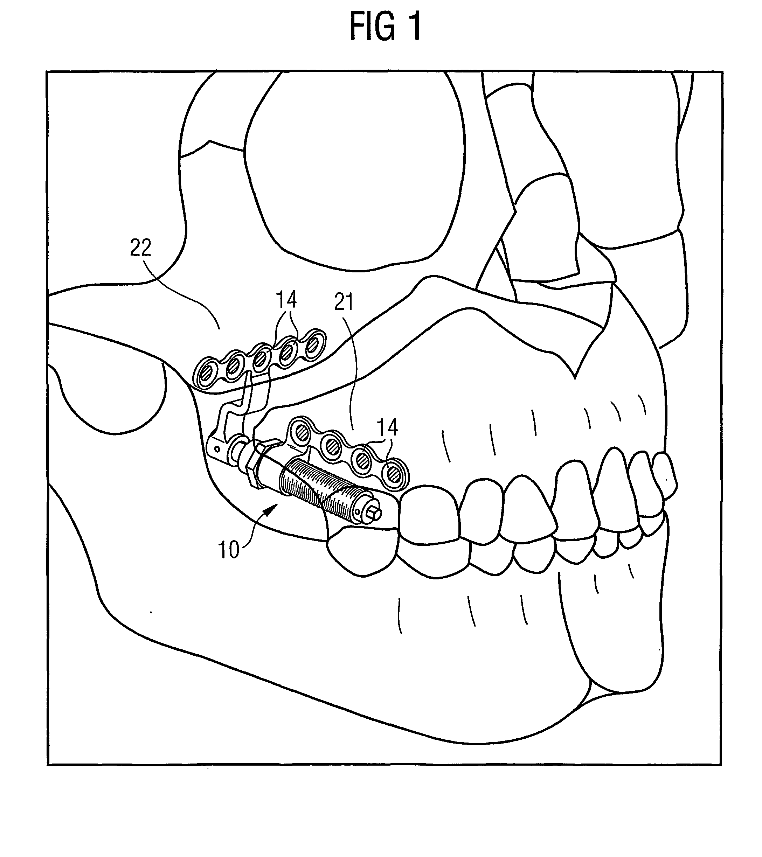

As seen in FIGS. 1, 2, and 3A, the orthopedic system 10 generally consists of distraction assembly 11 and anchors in the form of proximal and distal bone plates 500 and 700, respectively. The distraction assembly 11 has a proximal, or adjustment end 12 and a distal end 13. The orthopedic system 10 is affixed to maxilla 21 and zygoma 22 by bone screws 14 which are inserted though screw-holes 15 in footplates 500 and 700. In use, the entire orthopedic system 10 is implanted so that the distal bone plate 700 is attached to the zygoma 22 and the proximal bone plate 500 is attached to the maxilla 21, with the distraction assembly 11 nestled within the buccal sulcus. It will be understood that with reference to the various elements of the present invention, the term proximal is used to refer to the end of the device associated with the proximal end of the distraction assembly 12 that extends outwards away from the patient's zygoma 22. The term distal is used to refer to the other end of the device 13.

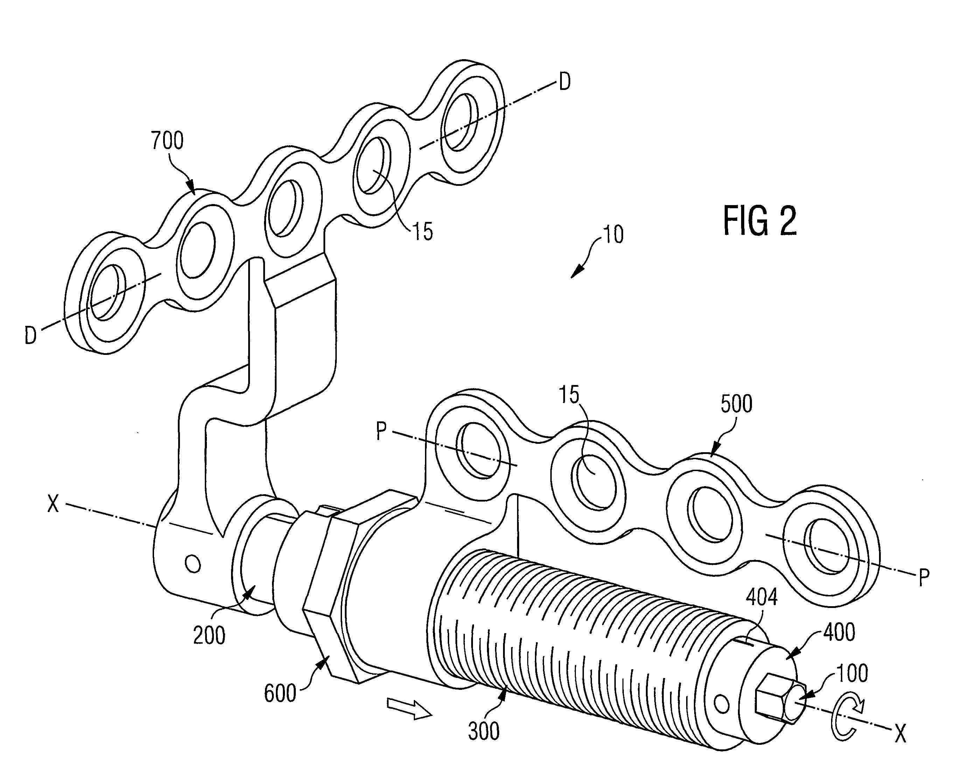

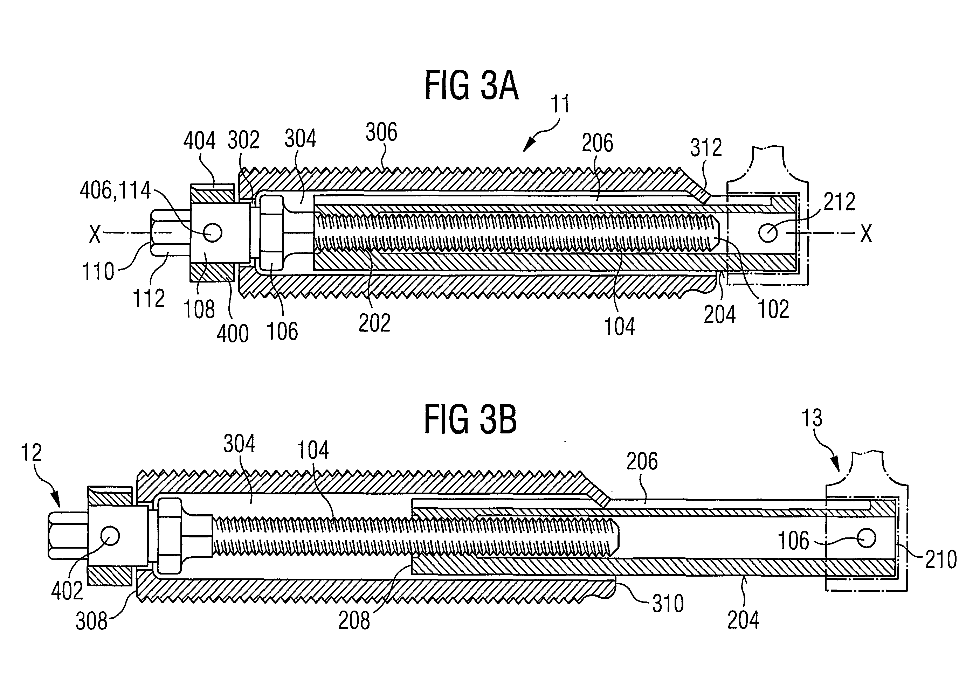

Turning now to the details of the orthopedic system 10 as best illustrated in FIGS. 2, 3A, and 3B, the distraction assembly 11 generally consists of a lead screw 100, an inner sleeve 200, and an outer sleeve 300. As described in detail below, lead screw 100 is journaled within outer sleeve 300, such that screw 100 can rotate, but not translate linearly (axially), relative to outer sleeve 300. Inner sleeve 200 has internal threading 202 which interacts with the external threading 104 on screw 100. Conversely, the interaction of the inner and outer sleeves, as discussed below, is such that they can translate linearly with respect to each other, but cannot rotate relative to each other. Thus, in the assembled distraction assembly 11, rotation of lead screw 100 is translated into linear motion of the inner sleeve 200 relative to the lead screw and outer sleeve, like a nut being driven on a bolt, causing telescopic expansion or contraction of the overall assembly 10.

Lead screw 100 has a distal shaft portion 102 provided with external screw threading 104, an enlarged-diameter intermediate portion 106, a proximal shaft portion 108, and a proximal, or adjustment end 110. Adjustment end 110 is provided with a tool interface 112, such as a hexagonal surface that can be driven by a standard hexagonal driving tool.

Inner sleeve 200 is provided with internal threading 202 along at least part of its length. The internal threading matches the external threading 104 on screw 100. The inner sleeve 200 has an exterior surface 204 which is generally smooth except for longitudinal slot 206 which extends from the proximal end 208 of the sleeve towards the distal end 210.

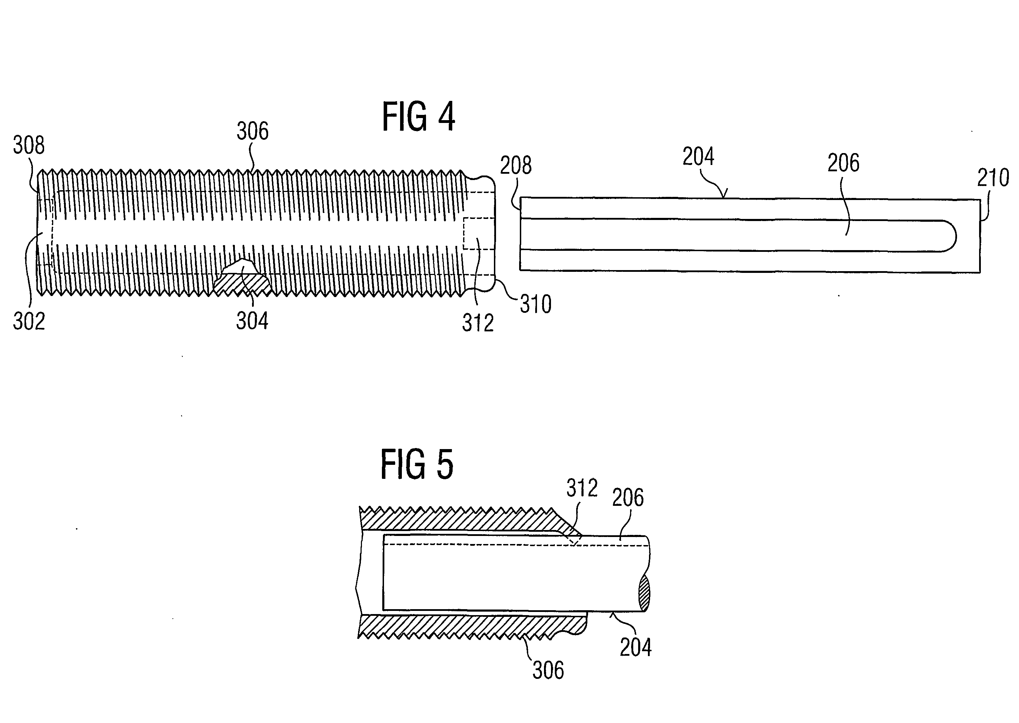

As best seen in FIG. 4, the outer sleeve 300 has two different inside cavity portions. The proximal cavity portion 302 has an inside diameter sized so as to (rotatably) slidably accept the proximal shaft portion 108 of the screw 100. The distal cavity portion 304 has an inside diameter sized so as to (axially) slidably accept the inner sleeve 200. The external surface of the outer sleeve 306 is preferably threaded along most of its length except for the distal end 310.

A mechanism is provided to prevent rotation but allow translation of the inner sleeve 200 in relation to the outer sleeve 300. In the illustrated embodiment, this is accomplished by having a portion of the distal end of the outer sleeve 310 formed into a "key" 312 which is sized to fit the longitudinal slot 206 of inner sleeve 200. This can be accomplished by crimping the distal end by application of a force, by an appropriately-shaped tool, sufficient to permanently deform a portion of the distal end. Alternatively, a pin could be fixed in a though hole in the wall of the distal end, flush with the outer surface and extending radially inward, the inner end fitting in the longitudinal slot 206.

Lead screw 100 is journaled withing the outer sleeve 300, so as to allow rotation of the lead screw 100 in relation to outer sleeve 300 but preventing translational motion. In a preferred embodiment, the journaling is accomplished according to the following. The proximal shaft portion 108 of lead screw 100 is slidably received within the proximal cavity portion 302 of the outer sleeve 300, such that screw 100 is free to rotate relative to the outer sleeve 300. A region of the proximal shaft portion 108, and the adjustment end 110 of screw 100, extend out from the proximal end 308 of the sleeve. A collar 400 is attached to the screw on the extending region of the proximal shaft portion, for example, by inserting pin 402 through matching holes 406 and 114 in the collar and proximal shaft portion, respectively. The collar 400 and the enlarged-diameter intermediate shaft portion 106 "sandwich" the proximal end 308 of outer sleeve 300, thereby preventing axial translation of the screw 100 relative to outer sleeve 300. In this way, screw 100 is effectively journaled within the outer sleeve 300.

The collar 400 also has a marking, such as an indentation, that acts as a visual reference mark 404. Since the collar rotates in conjunction with the lead screw , the reference mark 404 gives the user of the device an easily usable visual means to measure the amount of rotation that the lead screw undergoes when it is adjusted. Knowing the thread pitch of the device, the user can easily convert angular displacement of the mark into linear advancement of the device. Other visual marking methods can be used, including the imprinting of marks on the surface of the collar.

The internal threading 202 of inner sleeve 200 interacts with the external screw threading 104 of the lead screw 100, while at the same time the smooth exterior surface 204 of the inner sleeve is in sliding relation with the smooth inner surface of the distal cavity portion 304 of the outer sleeve. FIG. 5 illustrates how key 312 of the outer sleeve interacts with the longitudinal slot 206 to form a keyway. It will be appreciated that the interaction of longitudinal slot 206 and key 312 form a keyway which prevents relative rotation of the sleeves about the longitudinal axis X-X of the device (designated X-X in FIG. 3), while freely permitting sliding, telescoping movement of the inner sleeve 200 relative to the outer sleeve 300.

The system provides a mechanism whereby the distractor is anchored or affixed to the patient, for example, by proximal and distal footplates 500 and 700, which are best understood by reference to FIG. 2. The footplates are provided with screw holes 15 to accept the bone screws 14 which affix the device to the bone on either side of the patient's bone repair site. These holes are preferably countersunk to reduce the height of projection of the screw heads above the footplate surface after the device is fully implanted. The footplates have bottom, coupling surfaces 506 and 710 which may be flat or preferably may be shaped to conform to the contours of the bone to which it is being attached. As discussed in detail below, the coupling surfaces are bone-contacting surfaces when the footplates are attached directly to the patient's bone, or may be construct-contacting surfaces when the footplate is attached to a construct which is in turn mechanically coupled to the patient's bone.

Footplates 500 and 700 serve as the anchors, and can be made from any biocompatible material such as metal, plastic, or composites. For example, the footplates may be made of titanium or titanium alloy. The choice of material from which to construct the footplates is a routine design matter which depends purely on the particular medical application in which the system according to this invention is used. In a preferred embodiment, the footplates are bone plates made of stainless steel. Experiments have shown that stainless steel provides the necessary strength while at the same time being malleable enough to (i) allow for adjustments to the footplates by bending them, and (ii) withstand the cyclic loading inherent in the jaw area.

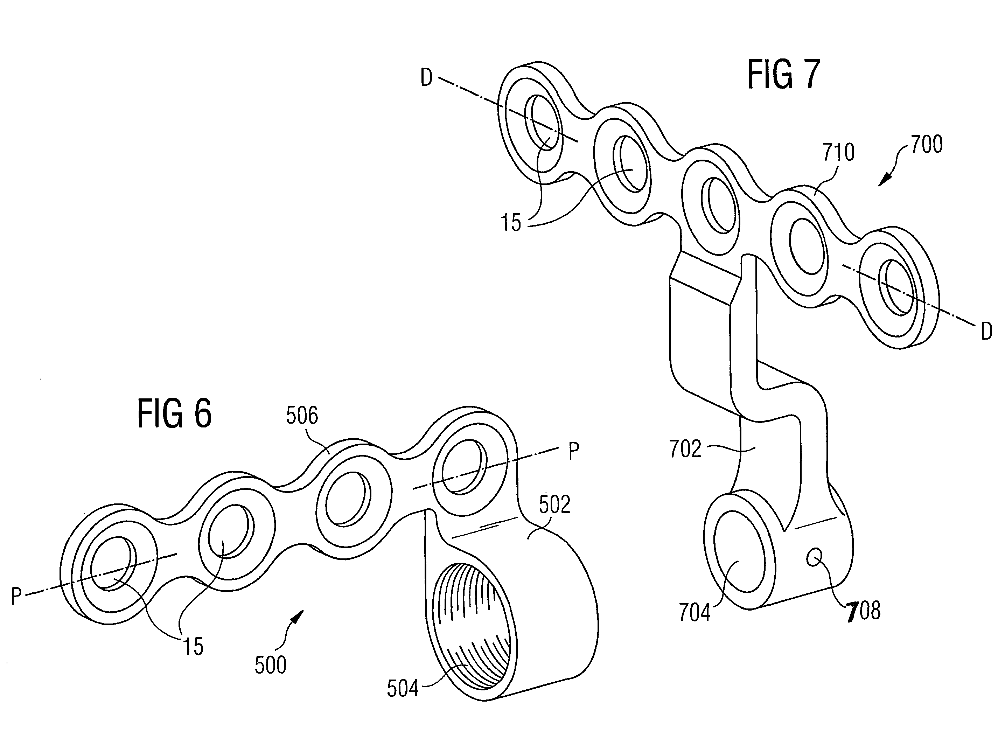

As shown in FIG. 6, the proximal footplate 500 has a device-connecting portion 502 comprising an internally-threaded bore 504 which accepts the threading on the external surface 306 of the outer sleeve 300. The internally-threaded bore of proximal footplate 504 interacts with the external surface 306 of outer sleeve 300, so that the orientation and separation of the two footplates in relation to each other can be modified as needed, by screwing the sleeve 300 into the bore 504. Once the desired orientation and separation is achieved, proximal footplate 500 is locked into position by tightening locking nut 600 against it, providing sufficient frictional force to keep the footplate in place.

As shown in FIG. 7, the distal footplate 700 has a device-connecting portion 702 comprising a bore 704 with a diameter that will accept inner sleeve 200. The distal footplate is attached to the distal end of inner sleeve 210, for example, by pressing the two together, and inserting a pin through holes 708 and 212.

As best illustrated in FIG. 2, the proximal footplate 500 is oriented so that line P-P is generally parallel to axis X-X of the distraction assembly 11. It is also offset above and to either side of the distraction assembly 11, depending on which side of the patient the assembly is to be implanted. When placed on the right side of the patient, the footplate 500 is offset to the left of the distraction assembly 11, and vice-versa. FIG. 2 shows the right- side orientation of the footplate, while FIG. 6 shows the left-side orientation. The distal footplate 700 is oriented so that line D-D is generally orthogonal to and above axis X-X of the distraction assembly 11.

The above-described geometry of footplates 500 and 700 has been found to provide a good combination of accessibility to the screws and holding strength when the device of the present invention is used in the distraction of the maxilla. However, it is to be understood that the precise location of the screw holes and the contoured shape and orientation of plates 500 and 700 as seen in FIGS. 2, 6, and 7 are not a critical aspect of the invention; other screw hole placements, plate shapes, and plate orientations could be used without departing from the spirit and scope of the present invention.

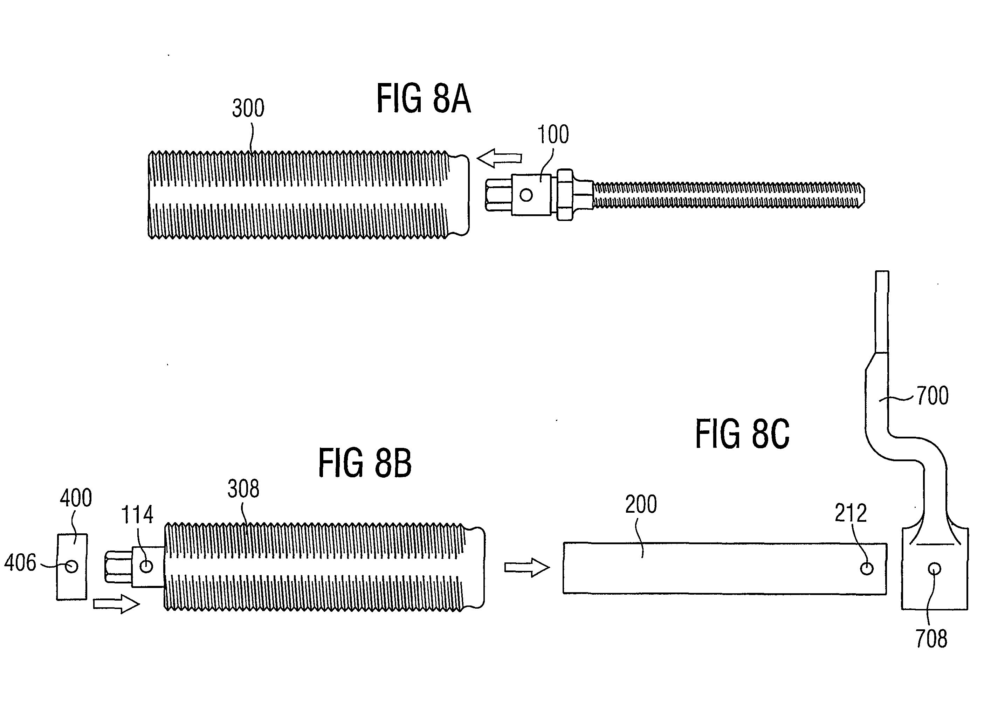

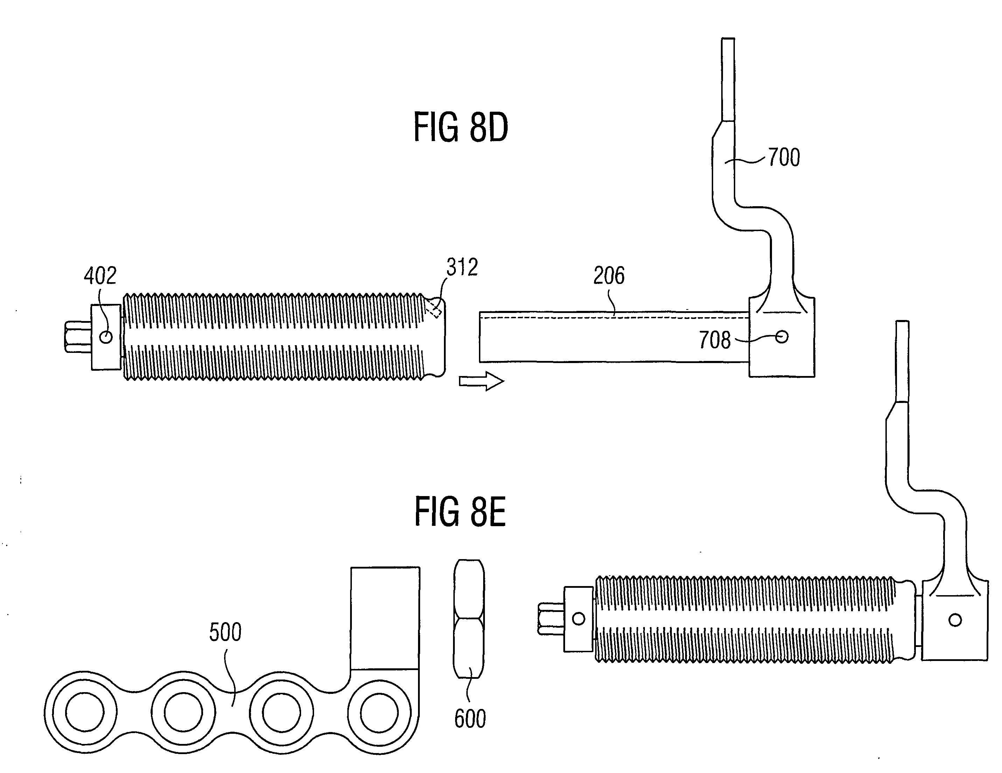

The assembly of the orthopedic system is best understood by reference to FIGS. 8A through 8E. To assemble the system, the lead screw 100 is first inserted into the outer sleeve 300, as shown in FIG. 8A. The collar 400 is then installed on the region of the proximal shaft portion 108 which extends out from the proximal end 308 of the outer sleeve 300, as shown in FIG. 8B. The collar 400 is captivated on the shaft by pressing pin 402 though matching holes in the collar 406 and proximal shaft portion 114. The distal footplate 700 is pressed onto the distal end of inner sleeve 210, as shown in FIG 8C, and captivated on the shaft by pressing pin 706 through matching holes in the footplate 708 and the distal shaft portion 212. The lead screw is then threaded into inner sleeve 200, as shown in FIG. 8D, care being taken that the longitudinal slot 206 on sleeve 200 is properly engaging key 312. Nut 600 and proximal footplate 500 are then threaded onto outer sleeve 300, as shown in FIG. 8E.

The device of the present invention is normally used in pairs, one for each side of the patient's face. In order to use the device of the present invention in a maxillary distraction procedure, the surgeon makes an incision, fits the devices to the patient, temporarily removes the devices in order to perform a LeFort I osteotomy (the separation of the maxilla from the rest of the midface), attaches the devices, performs distraction and consolidation, then permanently removes the devices.

To implant the device, a maxillary vestibular incision is made on the side of the patient's mouth, so that the periostium can be elevated to expose the maxillary and zygomatic bone. The assembled device is placed in the proper location and checked for the proper fit. Although the footplates are generally pre-shaped to be oriented in the proper manner, adjustments can be made to the footplates by bending them, for example, with a set of pliers. However, this deformability of the footplates is not an alternative to the means according to the invention for changing the orientation of the footplates.
The distal footplate is then fastened to the zygoma with bone screws 14, using a number sufficient to provide the necessary stability and strength. In a preferred method, the screws are self-tapping, so no pre-tapping of the bone is required. If needed, excess material in the footplate can be removed. For example, if not all of the screw holes need to be used, the portion of the footplate having the unused holes may be clipped off. The anterior footplate is then attached in the same manner. The same procedure is then repeated on the other side of the patient.
The doctor then sketches out the planned osteotomy (typically a LeFort I osteotomy), making allowances for the distraction devices. The devices are removed, the osteotomy is performed, and the devices are put back into place. The incision is then closed, leaving the distraction assemblies exposed, but within the patient's mouth.

The distraction osteogenesis procedure is performed by turning the lead screws on each device using the tool interface 112. It will be understood by reference to FIG. 1 (which does not illustrate soft tissue) that the distal end of the devices, where tool interface 112 is found, is easily accessible in the intra-oral region, between the patient's cheek and gum. Counter-clockwise rotation of the screw will result in axial lengthening of the device, resulting in a distraction force being communicated to the bones through the footplates. The reference mark 404 can be used to measure the changes in advancement precisely. Generally, distraction progresses at a rate of 1-2mm per day until full advancement is achieved. The advancement phase is followed by a consolidation phase, with a duration of at least twice as long as that of the advancement phase. The devices are then removed in a separate surgical procedure.

In a method not forming part of the invention, the proximal footplates 500 of the devices are not attached to the patient's maxilla 21, but rather to a construct, such as a dental splint, which is attached to the maxilla 21. A typical dental splint may consist of a disk of acrylic fitted or wired to the patient's teeth. Except for the differences described, this alternative method of treatment is the same as that used in the normal course of treatment. This embodiment can be used when the maxilla 21 of the patient cannot support the bone screws 14 used to support the footplates 500. This is often the case with cleft-lip or - palate patients, who often have large voids in the maxilla 21 where bone should be present. It may also be the preferred embodiment for treating younger patients, due to the presence of uninterrupted tooth buds which might be damaged by bone screws 14.

It should be emphasized that the above described embodiments of methods to attach the device to the patient are merely specific examples for mechanically coupling the device to the zygoma and maxilla. The device footplates are attachable directly to the patient's bone. They may also be attachable to one or more constructs, which constructs are attached to the patient's bone. Indeed, the constructs do not necessarily need to be directly attached to the patient's maxilla, but rather may be attached to the patient's teeth. What is important is that the device is mechanically coupled to the zygoma and the maxilla with sufficient rigidity in order to reliably perform the distraction. In a method not forming part of the invention the device may be implanted using circummaxillary wiring, in which wire is passed around the bony structure of the maxilla, to provide a firm anchorage for the device.

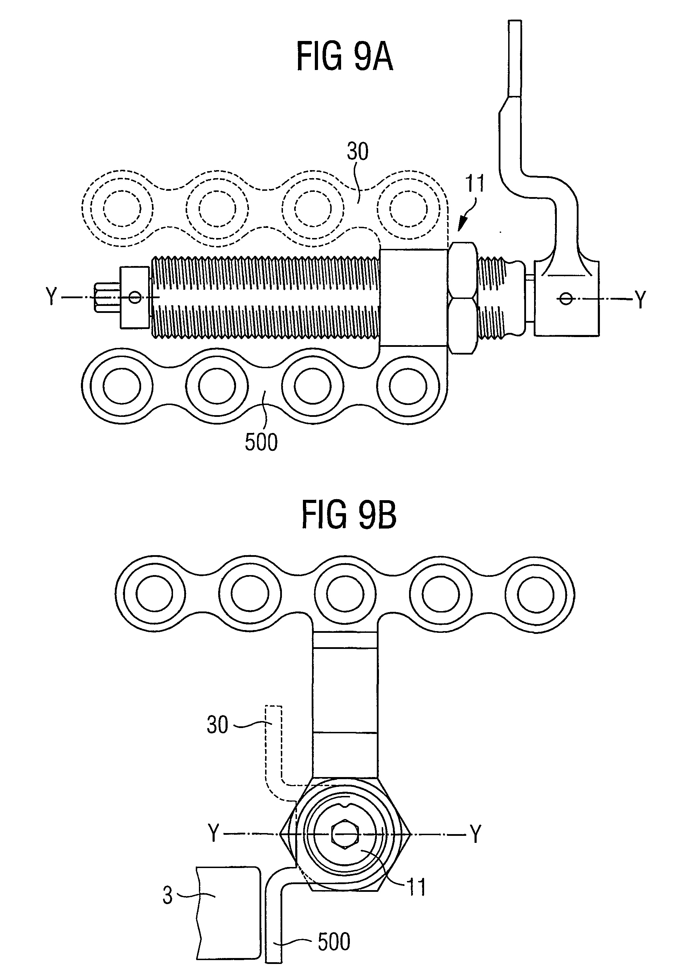

FIGS. 9A and 9B shows the device as it would be implanted on the left side of a patient using this embodiment. The orientation of the proximal footplates 500 is mirrored from its normal orientation 30 about the horizontal plane denoted by Y-Y. That is, for the device used on the left side of the patient, the footplate 500 is positioned below and to the right of the distraction assembly 11, as seen in FIGS. 9A and 9B. In practice, this may be done by simply rotating the footplate 500 one hundred eighty degrees (180) about the X-X axis (as described in FIG. 3A), and switching the side of the patient's face to which the device is implanted. Put another way, the footplate 500 used on the right side of the patient when attaching the device directly to the maxilla 21 is the same one used on the left side when attaching the device to a dental splint, and vice-versa. This orientation is preferred for the dental splint method because it places the footplate and screw holes closer to the horizontal plane created by the chewing surfaces of the teeth, which is the preferred position for attachment of the footplate to a dental splint. FIG. 9B shows a portion of the splint 3 in relation to the footplate 500.

In another preferred embodiment, the footplates and/or bone screws may be made from a bioresorbable material, and are detachable from the distraction assembly. This allows easy shaping of the footplates (when heated prior to insertion, for example by soaking in hot water). After distraction and consolidation have been completed, the bioresorbable footplates are detached from the distraction assembly and the incisions are closed, leaving the footplates and bone screws in place, to eventually be absorbed into the body. This provides the advantage of not having to perform a second surgical procedure to access the screws to remove the footplates. By reducing the number of surgical procedures required, the unavoidable risk and possible complications associated with any surgery is reduced. The bone screws should be made of a material that takes at least as long to absorb as the material the footplates are made of, thus ensuring that the footplates are secured until absorbed fully by the body.

It should be emphasized that the above described embodiments of the present invention are merely specific examples adapted for specific application in the human skeletal system. The modifications appropriate for other applications may readily be realized by those who are skilled in the art and who have been equipped with the understanding of the structure and operation of the present invention as set forth in the above description.

## Claims

1. An orthopedic system (10) for modifying the distance between the maxilla (21) and the zygoma (22) of a patient, **characterized in that** the system comprises:
A) a distal footplate (700) for subcutaneous implantation and attachment to the zygoma (22), the distal footplate (700) having a surface for coupling to bone and screw holes (15) adapted to receive bone screws (14);
B) a proximal footplate (500) for subcutaneous implantation and attachment to the maxilla (21), the proximal footplate (500) having a surface for coupling to bone and screw holes (15) adapted to receive bone screws (14);
C) an orthopedic device (11) having a longitudinal axis, the orthopedic device (11) allowing modification of the distance along the longitudinal axis between the footplates (500,700) once the orthopedic device (11) is implanted in the patient, the orthopedic device (11) further allowing modification of the orientation of the footplates (500,700) in relation to each other before implantation; whereby
D) said modification of the distance being effectable by action of said orthopedic device (11) being designed as a distractor or distraction assembly to that purpose
E) said modification of the orientation being effectable in the assembled state by means of a threaded connection between a footplate and the orthopedic device (11).

2. The orthopedic system (10) of claim 1, wherein the footplates (500,700) are bendable but still withstanding the cyclic loading inherent in the jaw area.

3. The orthopedic system (10) of claim 1 or 2, wherein the distal footplate (700) is attachable to the patient's zygoma (22) and the proximal footplate (500) is attachable to a construct, the construct being mechanically coupled to the patient's teeth.

4. The orthopedic system (10) according to one claim 2 or 3, wherein the footplates (500,700) can be detached from the distraction assembly.

5. The orthopedic system (10) according to one of the claims 1 to 4, wherein when the footplates (500,700) are engaged with the orthopedic device (11), the coupling surface of the distal footplate (700) is substantially orthogonal to the longitudinal axis of the orthopedic device (11) and the coupling surface of the proximal footplate (500) is substantially parallel to the longitudinal axis of the orthopedic device (11).

6. The orthopedic system (10) according to one of the claims 1 to 5, wherein the orthopedic device (11) comprises:
a) a lead screw (100) having external threading;
b) an inner sleeve (200) having an internal thread engaging the external thread of the lead screw (100); and an external axial slot; and a distal footplate engagement portion, the distal footplate engagement portion being coupled to the distal footplate (700); and
c) an outer sleeve (300) having a slot-engaging portion and a proximal footplate engagement portion, the proximal footplate engagement portion being coupled to the proximal footplate (500);
wherein the lead screw (100) and the outer sleeve (300) are joined such that only relative rotational movement about the longitudinal axis is allowed, and the inner sleeve (200) interacts with the lead screw (100) and outer sleeve (300) such that rotation of the lead screw (100) is translated into linear movement of the inner sleeve (200) in relation to the outer sleeve (300) and lead screw (100).

7. The orthopedic system (10) according to claim 6, wherein the slot-engaging portion of the outer sleeve (300) is an indentation in the distal end of the outer sleeve (300), extending radially inward to interact with the external axial slot of the inner sleeve (200).

8. The orthopedic system (10) according to claim 6, wherein the slot-engaging portion of the outer sleeve (300) is a pin inserted through the distal end of the outer sleeve (300), extending radially inward to interact with the external axial slot of the inner sleeve (200).

9. The orthopedic system (10) according to claim 6, wherein the lead screw (100) is joined to the outer sleeve (300) by a collar that rotates in conjunction with the lead screw (100).

10. The orthopedic system (10) according to claim of claim 9, wherein the collar is joined to the lead screw (100) by means of a pin inserted through both parts.

11. The orthopedic system (10) according to claim 9, wherein the collar has a visual reference mark to aid in converting angular displacement of the lead screw (100) to linear advancement of the outer sleeve (300) in relation to the inner sleeve (200).

12. The orthopedic system (10) according to 11, wherein the visual reference mark is an indentation in the surface of the collar.

13. The orthopedic system (10) according to claim 11, wherein that the visual reference mark is imprinted onto the surface of the collar.

14. The orthopedic system (10) according to one of the claims 6 to 13, wherein the distal footplate (700) has a bore with internal diameter matching that of the inner sleeve (200), and is attached to the inner sleeve (200) by pressing the two together and inserting a pin through both parts.

15. The orthopedic system (10) according to one of the claims 6 to 14, wherein the proximal footplate (500) has a bore with internal thread matching an external thread on the outer sleeve (300), and is attached to the outer sleeve (300) of the device by screwing the proximal footplate (500) onto the outer sleeve (300).

16. The orthopedic system (10) according to claim 15, further comprising a locking nut engaging the external thread of the outer sleeve (300), and wherein the proximal footplate (500) is locked into place by tightening the nut against it.

17. The orthopedic system (10) according to one of the claims 1 to 16 wherein the footplates (500,700) are made of a bioresorbable material.

18. The orthopedic system (10) according to one of the claims 1 to 16, wherein the footplates (500,700) are made of stainless steel.

19. The orthopedic system (10) according to one of the claims 1 to 16, wherein the footplates (500,700) are made of a material selected from the group of titanium or titanium alloy.

## Revendications

1. Système orthopédique (10) permettant de modifier l'écart entre le maxillaire (21) et le zygoma (22) d'un patient, **caractérisé en ce que** le système comprend :
A) une plaque d'assise distale (700) destinée à être implantée et mise en place de manière hypodermique sur le zygoma (22), la plaque d'assise distale (700) présentant une surface de mise en liaison avec l'os et des trous de passage de vis (15) adaptés à recevoir des vis d'ostéosynthèse (14);
B) une plaque d'assise proximale (500) destinée à être implantée et mise en place de manière hypodermique sur le maxillaire (21), la plaque d'assise proximale (500) présentant une surface de mise en liaison avec l'os et des trous de passage de vis (15) adaptés à recevoir des vis d'ostéosynthèse (14);
C) un dispositif orthopédique (11) présentant un axe longitudinal, le dispositif orthopédique (11) permettant de modifier l'écart entre les plaques d'assise (500, 700) le long de l'axe longitudinal lorsque le dispositif orthopédique (11) est implanté dans le corps du patient, et le dispositif orthopédique (11) permettant en outre de modifier, avant l'implantation, l'orientation des plaques d'assise (500, 700) l'une par rapport à l'autre;
D) la modification de l'écart pouvant être réalisée en actionnant le dispositif orthopédique (11) qui est conçu, à cet effet, sous forme d'écarteur ou unité d'écartement;
E) la modification de l'orientation à l'état monté pouvant être réalisée au moyen d'une liaison par vissage entre une plaque d'assise et le dispositif orthopédique (11).

2. Système orthopédique (10) selon la revendication 1, les plaques d'assise (500, 700) étant pliables mais étant néanmoins capables de résister à la charge cyclique apparaissant de manière inhérente dans la zone de la mâchoire.

3. Système orthopédique (10) selon la revendication 1 ou 2, la plaque d'assise distale (700) pouvant être mise en place sur le zygoma (22) du patient et la plaque d'assise proximale (500) pouvant être mise en place sur un élément structurel, ledit élément structurel étant lié de manière mécanique aux dents du patient.

4. Système orthopédique (10) selon la revendication 2 ou 3, les plaques d'assise (500, 700) pouvant être détachées de l'unité d'écartement.

5. Système orthopédique (10) selon l'une des revendications 1 à 4, la surface de liaison de la plaque d'assise distale (700) s'étendant essentiellement orthogonalement à l'axe longitudinal du dispositif orthopédique (11) et la surface de liaison de la plaque d'assise proximale (500) s'étendant essentiellement parallèlement à l'axe longitudinal du dispositif orthopédique (11), lorsque les plaques d'assise (500, 700) sont en prise avec le dispositif orthopédique (11).

6. Système orthopédique (10) selon l'une des revendications 1 à 5, le dispositif orthopédique (11) comprenant :
a) une vis conductrice (110) pourvue d'un filetage extérieur;
b) une douille intérieure (200) pourvue d'un filetage intérieur en prise avec le filetage extérieur de la vis conductrice (100); et d'une fente extérieure axiale; et d'une partie destinée à être engagée dans la plaque d'assise distale, la partie destinée à être engagée dans la plaque d'assise distale étant reliée à la plaque d'assise distale (700); et
c) une douille extérieure (300) présentant une partie destinée à être engagée dans la fente et une partie destinée à être engagée dans la plaque d'assise proximale, la partie destinée à être engagée dans la plaque d'assise proximale étant reliée à la plaque d'assise proximale (500); la vis conductrice (100) et la douille extérieure (300) étant reliées l'une à l'autre de manière à permettre uniquement un mouvement de rotation relatif autour de l'axe longitudinal, et la douille intérieure (200) étant en interaction avec la vis conductrice (100) et la douille extérieure (300) de telle sorte qu'une rotation de la vis conductrice (100) est transformée en un mouvement linéaire de la douille intérieure (200) par rapport à la douille extérieure (300) et à la vis conductrice (100).

7. Système orthopédique (10) selon la revendication 6, la partie de la douille extérieure (300) destinée à être engagée dans la fente étant une dent formée dans l'extrémité distale de ladite douille extérieure (300) et qui s'étend radialement vers l'intérieur afin d'entrer en interaction avec la fente extérieure axiale de la douille intérieure (200).

8. Système orthopédique (10) selon la revendication 6, la partie de la douille extérieure (300) destinée à être engagée dans la fente étant une cheville insérée dans l'extrémité distale de ladite douille extérieure (300) et qui s'étend radialement vers l'intérieur afin d'entrer en interaction avec la fente extérieure axiale de la douille intérieure (200).

9. Système orthopédique (10) selon la revendication 6, la vis conductrice (100) étant reliée à la douille extérieure (300) par une collerette qui tourne ensemble avec la vis conductrice (100).

10. Système orthopédique (10) selon la revendication 9, la collerette étant reliée à la vis conductrice (100) par une cheville qui est insérée à travers les deux éléments.

11. Système orthopédique (10) selon la revendication 9, la collerette présentant un marquage de référence visuel qui aide à convertir le déplacement angulaire de la vis conductrice (100) en un avancement linéaire de la douille extérieure (300) par rapport à la douille intérieure (200).

12. Système orthopédique (10) selon la revendication 11, le marquage de référence visuel étant une impression en creux dans la surface de la collerette.

13. Système orthopédique (10) selon la revendication 11, le marquage de référence visuel étant imprimé sur la surface de la collerette.

14. Système orthopédique (10) selon l'une des revendications 6 à 13, la plaque d'assise distale (700) présentant un trou avec un diamètre intérieur correspondant à celui de la douille intérieure (200), et ladite plaque étant mise en place sur la douille intérieure (200) en pressant les deux l'une contre l'autre et en insérant une cheville à travers les deux éléments.

15. Système orthopédique (10) selon l'une des revendications 6 à 14, la plaque d'assise proximale (500) présentant un trou pourvu d'un filetage intérieur correspondant au filetage extérieur de la douille extérieur (300), et ladite plaque étant mise en place sur la douille extérieure (300) du dispositif en vissant la plaque d'assise proximale (500) sur la douille extérieure (300).

16. Système orthopédique (10) selon la revendication 15, lequel comprend en outre un écrou de serrage en prise avec le filetage extérieur de la douille extérieure (300) et la plaque d'assise proximale (500) étant bloquée dans la position souhaitée en serrant l'écrou de serrage contre celle-ci.

17. Système orthopédique (10) selon l'une des revendications 1 à 16, les plaques d'assise (500, 700) étant fabriquées en un matériau biorésorbable.

18. Système orthopédique (10) selon l'une des revendications 1 à 16, les plaques d'assise (500, 700) étant fabriquées en acier inoxydable.

19. Système orthopédique (10) selon l'une des revendications 1 à 16, les plaques d'assise (500, 700) étant fabriquées en un matériau choisi dans le groupe comprenant le titane ou l'alliage de titane.

## Patentansprüche

1. Orthopädisches System (10) zur Veränderung des Abstands zwischen der Maxilla (21) und dem Zygoma (22) eines Patienten, **dadurch gekennzeichnet, dass** das System folgendes umfasst:
A) eine distale Fussplatte (700) zur subkutanen Implantation und Anbringung an das Zygoma (22), wobei die distale Fussplatte (700) eine Knochenverbindungsfläche und zur Aufnahme von Knochenschrauben (14) angepasste Schraubenlöcher (15) aufweist;
B) eine proximale Fussplatte (500) zur subkutanen Implantation und Anbringung an die Maxilla (21), wobei die proximale Fussplatte (500) eine Knochenverbindungsfläche und zur Aufnahme von Knochenschrauben (14) angepasste Schraubenlöcher (15) aufweist;
C) eine orthopädische Vorrichtung (11) mit einer Längsachse, wobei die orthopädische Vorrichtung (11) eine entlang der Längsachse erfolgende Veränderung des Abstands zwischen den Fussplatten (500, 700) erlaubt, wenn die orthopädische Vorrichtung (11) im Körper des Patienten implantiert ist, und
wobei die orthopädische Vorrichtung (11) weiterhin eine vor der Implantation erfolgende Veränderung der Ausrichtung der Fussplatten (500, 700) in Bezug aufeinander erlaubt; wobei
D) die Veränderung des Abstands durch Betätigen der orthopädischen Vorrichtung (11) durchführbar ist, welche zu diesem Zweck als Distraktor bzw. Distraktionseinrichtung ausgelegt ist;
E) die Veränderung der Ausrichtung in montiertem Zustand mittels einer Gewindeverbindung zwischen einer Fussplatte und der orthopädischen Vorrichtung (11) durchführbar ist.

2. Orthopädisches System (10) nach Anspruch 1, wobei die Fussplatten (500, 700) biegbar sind, jedoch dennoch der im Kieferbereich inhärent auftretenden zyklischen Belastung standhalten.

3. Orthopädisches System (10) nach Anspruch 1 oder 2, wobei die distale Fussplatte (700) an das Zygoma (22) des Patienten anbringbar ist und die proximale Fussplatte (500) an ein Strukturelement anbringbar ist, wobei das Strukturelement auf mechanische Weise mit den Zähnen des Patienten verbunden ist.

4. Orthopädisches System (10) nach Anspruch 2 oder 3, wobei die Fussplatten (500, 700) von der Distraktionseinrichtung abgenommen werden können.

5. Orthopädisches System (10) nach einem der Ansprüche 1 bis 4, wobei wenn die Fussplatten (500, 700) mit der orthopädische Vorrichtung (11) in Eingriff stehen, sich die Verbindungsfläche der distalen Fussplatte (700) im wesentlichen orthogonal zu der Längsachse der orthopädischen Vorrichtung (11) erstreckt und sich die Verbindungsfläche der proximalen Fussplatte (500) im wesentlichen parallel zu der Längsachse der orthopädischen Vorrichtung (11) erstreckt.

6. Orthopädisches System (10) nach einem der Ansprüche 1 bis 5, wobei die orthopädische Vorrichtung (11) folgendes umfasst:
a) eine Leitspindel (110) mit einem Aussengewinde;
b) eine Innenhülse (200) mit einem mit dem Aussengewinde der Leitspindel (100) in Eingriff stehenden Innengewinde; und mit einen äusseren, axialen Schlitz; und mit einem Eingriffsabschnitt der distalen Fussplatte, wobei der Eingriffsabschnitt der distalen Fussplatte mit der distalen Fussplatte (700) verbunden ist; und
c) eine Aussenhülse (300) mit einem Schlitz-Eingriffsabschnitt und mit einem Eingriffsabschnitt der proximalen Fussplatte auf, wobei der Eingriffsabschnitt der proximalen Fussplatte mit der proximalen Fussplatte (500) verbunden ist;
wobei die Leitspindel (100) und die Aussenhülse (300) so miteinander verbunden sind, dass nur eine relative Rotationsbewegung um die Längsachse ermöglicht wird, und die Innenhülse (200) mit der Leitspindel (100) und der Aussenhülse (300) in einer Weise in Wechselwirkung steht, dass ein Drehen der Leitspindel (100) in eine lineare Bewegung der Innenhülse (200) in Bezug auf die Aussenhülse (300) und die Leitspindel (100) umgesetzt wird.

7. Orthopädisches System (10) nach Anspruch 6, wobei es sich bei dem Schlitz-Eingriffsabschnitt der Aussenhülse (300) um eine in dem distalen Ende der Aussenhülse (300) ausgebildete Verzahnung handelt, welche sich radial nach innen erstreckt, um mit dem äusseren axialen Schlitz der Innenhülse (200) in Wechselwirkung zu treten.

8. Orthopädisches System (10) nach Anspruch 6, wobei es sich bei dem Schlitz-Eingriffsabschnitt der Aussenhülse (300) um einen durch das distale Ende der Aussenhülse (300) eingeführten Stift handelt, der sich radial nach innen erstreckt, um mit dem äusseren, axialen Schlitz der Innenhülse (200) in Wechselwirkung zu treten.

9. Orthopädisches System (10) nach Anspruch 6, wobei die Leitspindel (100) durch einen Kragen mit der Aussenhülse(300) verbunden ist, welcher sich zusammen mit der Leitspindel (100) dreht.

10. Orthopädisches System (10) nach Anspruch 9, wobei der Kragen durch einen Stift mit der Leitspindel (100) verbunden ist, welcher durch beide Teile hindurchgeführt wird .

11. Orthopädisches System (10) nach Anspruch 9, wobei der Kragen eine visuelle Referenzmarkierung aufweist, die bei der Konvertierung der Winkelverschiebung der Leitspindel (100) in einen linearen Vorschub der Aussenhülse (300) in Bezug auf die Innenhülse (200) behilflich ist.

12. Orthopädisches System (10) nach Anspruch 11, wobei es sich bei der visuellen Referenzmarkierung um eine Eindrückung in die Oberfläche des Kragens handelt.

13. Orthopädisches System (10) nach Anspruch 11, wobei die visuelle Referenzmarkierung auf die Oberfläche des Kragens aufgedruckt ist.

14. Orthopädisches System (10) nach einem der Ansprüche 6 bis 13, wobei die distale Fussplatte (700) eine Bohrung mit einem Innendurchmesser aufweist, welcher jenem der Innenhülse (200) entspricht, und sie an der Innenhülse (200) angebracht wird, indem die beiden zusammengepresst werden und ein Stift durch beide Teile hindurchgeführt wird.

15. Orthopädisches System (10) nach einem der Ansprüche 6 bis 14, wobei die proximale Fussplatte (500) eine Bohrung mit einem Innengewinde aufweist, welches dem Aussengewinde der Aussenhülse (300) entspricht, und sie an der Aussenhülse (300) der Vorrichtung angebracht wird, indem die proximale Fussplatte (500) auf die Aussenhülse (300) aufgeschraubt wird.

16. Orthopädisches System (10) nach Anspruch 15, welches weiterhin eine mit dem Aussengewinde der Aussenhülse (300) in Eingriff stehende Klemmmutter umfasst, und wobei die proximale Fussplatte (500) in der gewünschten Stellung festgeklemmt wird, indem die Mutter gegen diese festgezogen wird.

17. Orthopädisches System (10) nach einem der Ansprüche 1 bis 16, wobei die Fussplatten (500, 700) aus einem biologisch resorbierbaren Material gefertigt sind.

18. Orthopädisches System (10) nach einem der Ansprüche 1 bis 16, wobei die Fussplatten (500, 700) aus rostfreiem Stahl gefertigt sind.

19. Orthopädisches System (10) nach einem der Ansprüche 1 bis 16, wobei die Fussplatten (500, 700) aus einem Material gefertigt sind, welches aus der Gruppe bestehend aus Titan oder Titanlegierung ausgewählt ist.
